# EUROPEAN PATENT APPLICATION

(11) **EP 4 650 779 A1**
(43) Date of publication of application: **19.11.2025**
(21) Application number: 24741759.5
(22) Date of filing: 12.01.2024
(51) Int. Cl.: G01N 33/574, G01N 33/68, G01N 33/558

(54) **BIOMARKER FOR DIAGNOSIS OR PROGNOSIS OF SQUAMOUS CELL LUNG CARCINOMA USING EXOSOMES AND USE THEREOF**

(30) Priority: 13.01.2023 KR 20230005610
(71) Applicant: Korea University Research and Business Foundation, Seoul 02841 (KR)
(72) Inventor: KIM, Hyun Koo, Seoul 06579 (KR); CHOI, Byeong Hyeon, Seoul 08308 (KR); JEON, Ok Hwa, Seoul 08308 (KR); RHO, Ji-yun, Seoul 08096 (KR); KIM, Kyungsu, Seoul 03483 (KR); KIM, Chang Geun, Seoul 07628 (KR); KIM, Gayoon, Seoul 08014 (KR)
(74) Representative: Forresters IP LLP
(86) International application number: PCT/KR2024/000652
(87) International publication number: WO 2024/151138

(57) **Abstract**

The present invention relates to a biomarker composition including PBLD-overexpressing exosomes for diagnosing squamous cell lung carcinoma. According to the present invention, it is possible to diagnose subtypes of squamous cell lung carcinoma non-invasively, quickly, and accurately.

## Description

### TECHNICAL FIELD

The present disclosure relates to a biomarker for diagnosis or prognosis of squamous cell lung carcinoma using exosomes and uses thereof.

### BACKGROUND ART

Squamous cell lung carcinoma is lung cancer that originates from squamous cells and a type of non-small cell cancer that originates from squamous epithelial cells constituting the bronchial mucosa. The squamous epithelium is a flat-shaped epithelium, and the epithelium refers to a cell layer on the outer surface of the body and a cell layer surrounding the inner surface of the body cavity (a space between the body wall and the intestines) and the gastrointestinal tract. Squamous cell carcinoma is mainly found in the center of the lung, is more common in men, is highly related to smoking, and is reported to cause symptoms such as coughing, hemoptysis, and wheezing due to partial obstruction of the bronchial tubes.

A standard method for diagnosis of cancer is a tissue biopsy. When abnormal lesions are identified through a low-dose chest CT, tissues to be used for testing are obtained using tools such as needles under thoracoscopy or PET/CT imaging. However, such an invasive biopsy causes pain to the patient, and problems such as bleeding may occur during a biopsy process. There is also a problem with the false positive of low-dose chest CT, but the false positive rate reported in the National Lung Screen Trial (NSLT) in the United States was 26.8%, and the false positive rate reported in Korea was approximately 14.8%, and thus a considerable number of false positive rates has been reported.

In addition, in some cases, repeated follow-up examinations are difficult due to problems such as high examination costs and radiation exposure, and a tissue biopsy may not be performed depending on a patient's condition. Another problem of the tissue biopsy is a problem on heterogeneity of different biological characteristics between tumor tissues or within tumor tissues, and thus information obtained from some tissues may be insufficient to establish appropriate treatment and surgical plans.

As a need for early diagnosis of various diseases including cancer is raised, a research trend is changing from traditional tissue collection-based tissue biopsy and imaging-based diagnostic methods such as tissue staining to a liquid biopsy using biomarkers. In this trend, research on biomarkers, which are molecular biological markers derived from DNA, RNA, metabolites, proteins, and protein fragments, has been actively conducted.

However, no biomarker used for diagnosis of lung cancer has been currently reported to have a particularly significant effect, and some cancer markers CEA, SCC-Ag, etc. that have been announced to have increased diagnostic significance in lung cancer among cancer markers originating from other carcinomas are applied and used to lung cancer, but the biomarkers are not widely used in clinical practice because diagnostic sensitivity is very low.

Therefore, the present inventors have completed and provided the invention of a novel biomarker capable of diagnosing cancer early and non-invasively, with high sensitivity and specificity, specifically for a squamous cell lung carcinoma subtype among lung cancers.

### DISCLOSURE OF THE INVENTION

### TECHNICAL GOALS

An aspect to be achieved by the present disclosure is to provide a biomarker composition for diagnosis or prognosis of squamous cell lung carcinoma, including exosomes overexpressing a phenazine biosynthesis-like domain-containing protein (PBLD, UniProt accession No. P30039) or a gene encoding the PBLD protein.

Another aspect to be achieved by the present disclosure is to provide a composition for diagnosis or prognosis of squamous cell lung carcinoma, including a preparation capable of measuring the expression level of a PBLD protein or a gene encoding the PBLD protein in exosomes.

However, technical goals to be achieved are not limited to those described above, and other goals not mentioned above are clearly understood by one of ordinary skill in the art from the following description.

### TECHNICAL SOLUTIONS

In order to solve the problems, the present inventors provide a biomarker composition for diagnosis or prognosis of squamous cell lung carcinoma, including exosomes overexpressing a phenazine biosynthesis-like domain-containing protein (PBLD, UniProt accession No. P30039) or a gene encoding the PBLD protein.

According to another embodiment of the present disclosure, there is provided a composition for diagnosis or prognosis of squamous cell lung carcinoma, including a preparation capable of measuring the expression level of a PBLD protein or a gene encoding the PBLD protein in exosomes.

According to one aspect, the preparation capable of measuring the expression level of the protein may be an antibody or an antigen-binding fragment thereof that specifically binds to the protein; or an aptamer that specifically binds to the protein.

According to one aspect, the preparation capable of measuring the expression level of the gene may be a primer or probe that specifically binds to a nucleic acid molecule of the gene.

According to yet another embodiment of the present disclosure, there is provided a method for providing information for diagnosis or prognosis of squamous cell lung carcinoma, the method including:
a) extracting exosomes from a biological sample isolated from a subject; and
b) measuring the expression level of a PBLD protein or a gene encoding the PBLD protein in the extracted exosomes.

According to one aspect, the method may further include
c) extracting exosomes from a biological sample isolated from a control group;
d) measuring the expression level of a PBLD protein or a gene encoding the PBLD protein in the exosomes; and
e) comparing the expression levels of the protein or the gene encoding the protein in the subject and the control group.

According to one aspect, when the expression level of the PBLD protein or the gene encoding the PBLD protein of the subject is higher than that of the control group, the subject may be judged to have developed squamous cell lung carcinoma or predicted to have a high risk of developing the squamous cell lung carcinoma.

According to one aspect, when the expression level of the PBLD protein or the gene encoding the PBLD protein of the subject is higher than that of the control group, the subject may be predicted to have a poor prognosis.

According to another aspect of the present disclosure, there is provided a kit for diagnosis of squamous cell lung carcinoma including the composition of any one of the diagnostic compositions.

According to yet another embodiment of the present disclosure, there is provided a screening method of a squamous cell lung carcinoma therapeutic agent including:
a) treating a candidate substance to a biological sample, cell line, or non-human animal model isolated from a subject;
b) extracting exosomes from the biological sample, cell line or non-human animal model treated with the candidate substance; and
c) measuring the expression level of a PBLD protein or a gene encoding the PBLD protein in the extracted exosomes.

According to one aspect, the screening method may further include, after the step,
d) selecting a candidate substance having a reduced expression level of the PBLD protein or the gene encoding the PBLD protein compared to a control sample that is not treated with the candidate substance.

### EFFECTS OF THE INVENTION

According to the present disclosure, it is possible to early diagnose or prognose cancer specifically or non-invasively for a squamous cell lung carcinoma subtype among lung cancers, with high sensitivity and specificity.

The effects of the present disclosure are not limited to the aforementioned effects, and other effects, which are not mentioned above, will be clearly appreciated by a person having ordinary skill in the art from the following description.

### BRIEF DESCRIPTION OF DRAWINGS

FIG. 1 illustrates a proteomic analysis workflow in the present disclosure.
FIG. 2 shows a volcano plot for differences in protein expression levels between a control group and a squamous cell lung carcinoma group.
FIG. 3 shows results of a PBLD ELISA assay performed by extracting exosomes from the blood of a normal group and a squamous epithelial cell patient group.
FIG. 4 shows results of ROC analysis using exosome PBLD.
FIG. 5 shows results of exosome PBLD ELISA performed on the blood of patients with lung adenocarcinoma and squamous cell lung carcinoma.
FIG. 6 illustrates results of ROC and AUC analysis for lung adenocarcinoma and squamous cell lung carcinoma.
FIG. 7 shows a recurrence-free survival curve confirmed by extracting exosomes from the blood of patients with squamous cell lung carcinoma.

### BEST MODE FOR CARRYING OUT THE INVENTION

The present inventors collected exosomes from a squamous cell lung carcinoma patient group and performed a proteomic analysis thereon to confirm a PBLD protein, which was significantly highly expressed in the squamous cell lung carcinoma patient group compared to a control group, and completed and intended to provide the invention of the PBLD protein as a biomarker and a composition for diagnosis or prognosis of squamous cell lung carcinoma.

The present inventors provide a biomarker composition for diagnosis or prognosis of squamous cell lung carcinoma, including exosomes overexpressing a phenazine biosynthesis-like domain-containing protein (PBLD, UniProt accession No. P30039) or a gene encoding the PBLD protein.

As used herein, the term "exosomes overexpressing the PBLD protein or the gene encoding the same" refer to exosomes expressing the PBLD protein or the gene encoding the PBLD protein at a high level compared to a PBLD expression level or a predetermined cutoff value of exosomes isolated from a normal person.

The exosomes are nano-sized (30 to 150 nm) small vesicles secreted by most cells. It is known that various types of proteins, genetic materials (DNA, mRNA, and miRNA), lipids, and the like derived from cells are included in the exosome and the phospholipid bilayer membrane. In addition, it has been reported that tissue-derived exosomes may be used to diagnose diseases by reflecting the condition of a tissue that secreted the exosomes.

Accordingly, the present inventors completed the present disclosure by confirming that cancer can be diagnosed or prognosed accurately and quickly when using a PBLD protein specifically expressed in exosomes derived from squamous cell lung carcinoma or a gene encoding the PBLD protein.

As used herein, the 'diagnosis' means predicting the occurrence of diseases and determining the risk or susceptibility to developing the diseases, or confirming the presence or characteristics of a pathological condition.

For the purposes of the present disclosure, the diagnosis refers to diagnosis of squamous cell lung carcinoma. The term "squamous cell lung carcinoma" used herein refers to lung cancer originating from squamous cells, and a type of non-small cell cancer originating from squamous epithelial cells that constitute the bronchial mucosa.

As used herein, the term 'diagnostic (bio)marker, (bio)marker for diagnosis or diagnostic marker' is a marker which may diagnose squamous cell lung carcinoma distinguished from a normal control group, and refers to a marker including a preparation or a composition included in the marker capable of distinguishing, from normal cells, the level of organic biomolecules such as polypeptides or nucleic acids (e.g., mRNA, etc.), lipids, glycolipids, glycoproteins, sugars (monosaccharides, disaccharides, oligosaccharides, etc.), which show an increase or decrease in cells having squamous cell lung carcinoma compared to the normal cells, or measuring the level of the marker.

As used herein, the term "prognosis" means determining recurrence, metastasis, drug responsiveness, or resistance of the corresponding subject after treatment of squamous cell lung carcinoma. The prognosis may include a concept of predicting not only whether the corresponding subject develops squamous cell lung carcinoma, but also whether the corresponding subject has a good prognosis for survival in the future, by measuring the expression level of PBLD in exosomes isolated from a sample of the subject.

According to yet another embodiment of the present disclosure, there is provided a composition for diagnosis or prognosis of squamous cell lung carcinoma, including a preparation capable of measuring the expression level of a PBLD protein or a gene encoding the PBLD protein in exosomes.

As used herein, the term "measuring the expression level" means measuring the presence, expression, or expression level of a specific protein (peptide) or a gene encoding the protein, and specifically, may be measuring the expression level of the PBLD protein or an mRNA or gene encoding the PBLD protein.

According to one aspect, the preparation capable of measuring the expression level of the protein may be an antibody or an antigen-binding fragment thereof that specifically binds to the protein; or an aptamer that specifically binds to the protein.

The term "antibody" used herein is a term known in the art and means a specific immunoglobulin directed against an antigenic site. For example, the antibody may specifically bind to the PBLD protein or a fragment thereof. The fragment means a protein fragment having one or more epitopes that may be recognized by antibodies against the protein, and may be, for example, an immunogenic fragment. The form of the antibody includes a polyclonal antibody, a monoclonal antibody, or a recombinant antibody, and includes all immunoglobulin antibodies. In addition, the antibody includes special antibodies such as humanized antibodies.

Whether the protein has been expressed in the biological sample may be confirmed using these antibodies by methods known in the art, such as enzyme linked immunosorbent assay (ELISA), radioimmunoassay (RIA), sandwich assay, Western blotting or immunoblotting on a polyacrylic gel, and the like.

The term "antibody fragment" as used herein means a polypeptide that does not have the structure of an intact antibody, peptide or protein, but has a specific antigen-binding site or binding domain directed against an antigenic site. The fragment includes a functional fragment of an antibody molecule other than a complete antibody having two light chains and two heavy chains. The functional fragment of the antibody molecule refers to a fragment having at least an antigen binding function, and may be Fab, F(ab'), F(ab')2, or Fv. The binding fragment may include at least 7 amino acids, for example, at least 9 amino acids, or at least 12 amino acids.

The analysis method for detecting the protein includes Western blotting, enzyme linked immunosorbent assay (ELISA), radioimmunoassay (RIA), radioimmunodiffusion, Ouchterlony immunodiffusion, rocket immunoelectrophoresis, tissue immunostaining, immunoassay, immunohistochemistry assay, immunoprecipitation assay, complement fixation assay, fluorescence activated cell sorter (FACS), protein chips, and the like, but is not limited thereto.

According to one aspect, the preparation capable of measuring the expression level of the gene may be a primer or probe that specifically binds to a nucleic acid molecule of the gene. The analysis method may be used with, for example, at least one selected from the group consisting of reverse transcription polymerase reaction (RT-PCR), competitive RT-PCR, real-time RT-PCR, RNase protection assay (RPA), Northern blotting, DNA chips, etc.

The term "primer" as used herein refers to a nucleic acid sequence having a free 3' hydroxyl group, and a nucleic acid sequence which may form base pairs with a template complementary to a specific base sequence and serves as a starting point for copying a template strand. The primer may initiate DNA synthesis in the presence of a reagent (i.e., DNA polymerase or reverse transcriptase) for polymerization and four different nucleoside triphosphates in appropriate buffer solution and temperature. For example, the occurrence of squamous cell lung carcinoma in a subject, and the like may be diagnosed by performing PCR amplification using sense and antisense primers with 7 to 50 nucleotide sequences as specific primers for the gene or mRNA encoding the PBLD protein to measure the amount of a desired product. The PCR conditions, and the lengths of sense and antisense primers may be appropriately selected according to techniques known in the art. The primers may have 10 to 100, 15 to 100, 10 to 80, 10 to 50, 10 to 30, 10 to 20, 15 to 80, 15 to 50, 15 to 30, 15 to 20, 20 to 100, 20 to 80, 20 to 50, or 20 to 30 nt.

The term "probe" used herein means a nucleic acid fragment such as RNA or DNA that may specifically bind to a target nucleic acid, for example, mRNA, and may be labeled so as to confirm the presence or absence, content, and expression level of a specific mRNA. The probe may be fabricated in the form of an oligonucleotide probe, a single stranded DNA probe, a double stranded DNA probe, an RNA probe, or the like. For example, the expression level of mRNA may be measured through the degree of hybridization by performing hybridization using a probe having a nucleic acid sequence complementary to a gene or mRNA encoding the PBLD protein, thereby diagnosing whether or not a subject develops squamous cell lung carcinoma. The selection and hybridization conditions of the probe may be appropriately selected according to techniques known in the art. The probe may have 10 to 100, 15 to 100, 10 to 80, 10 to 50, 10 to 30, 10 to 20, 15 to 80, 15 to 50, 15 to 30, 15 to 20, 20 to 100, 20 to 80, 20 to 50, or 20 to 30 nt.

The primer or probe may be chemically synthesized using a phosphoramidite solid support synthesis method or other well-known methods. In addition, these nucleic acid sequences may be modified by various methods known in the art. Examples of such modifications may include methylation, encapsulation, substitution of one or more homologs of natural nucleotides, or modifications between nucleotides, such as uncharged linkers (e.g., methyl phosphonate, phosphotriester, phosphoroamidate, carbamate, etc.) or charged linkers (e.g., phosphorothioate, phosphorodithioate, etc.). In addition, the primer or probe may be modified using labels that may directly or indirectly provide a detectable signal. Examples of such labels may include radioactive isotopes, fluorescent molecules, or biotin.

According to yet another embodiment of the present disclosure, there is provided a kit for diagnosis of squamous cell lung carcinoma, including a preparation capable of measuring the expression level of a PBLD protein or a gene encoding the PBLD protein.

For example, the kit may provide information for diagnosis of squamous cell lung carcinoma by obtaining a biological sample from a specimen containing a tissue in which the occurrence of squamous cell lung carcinoma is expected or suspected, and extracting exosomes from the biological sample to confirm the expression level of the corresponding protein in the exosomes by using a composition containing a PBLD protein detection agent. The kit may be an immunoassay kit.

A kit for diagnosing squamous cell lung carcinoma according to another embodiment of the present disclosure provides information used for diagnosis of squamous cell lung carcinoma, including a preparation for detecting a gene encoding the PBLD protein. For example, the kit may provide information for diagnosis of squamous cell lung carcinoma by obtaining a biological sample from a specimen containing a tissue in which the occurrence of squamous cell lung carcinoma is expected or suspected, extracting a nucleic acid encoding a PBLD protein extracting exosomes from the biological sample, and determining the level of expression of a nucleic acid encoding the corresponding protein in the sample using a composition including a preparation for detecting the nucleic acid (in the case of using a sample in which mRNA is reverse transcribed, cDNA and an agent for detecting cDNA are applied). The kit may be a DNA chip.

According to yet another embodiment of the present disclosure, there is provided a method for providing information for diagnosis or prognosis of squamous cell lung carcinoma, the method including: a) extracting exosomes from a biological sample isolated from a subject; and
b) measuring the expression level of a PBLD protein or a gene encoding the PBLD protein in the extracted exosomes.

The term "subject" as used herein means any organism that has developed or is likely to develop squamous cell lung carcinoma, and may include mammals such as dogs, cats, mice, rats, monkeys, cows, pigs, mini-pigs, livestock, and humans, but is not limited thereto.

The term "sample" as used herein means a material derived from the subject, and specifically, may include tissue, cells, whole blood, serum, plasma, saliva, sputum, cerebrospinal fluid, urine, etc., and desirably, may be at least one selected from the group consisting of blood, plasma, lung tissue biopsy, nasopharyngeal swab, sputum, and saliva, but is not limited thereto.

According to one aspect, the method may further include:
c) extracting exosomes from a biological sample isolated from a control group;
d) measuring the expression level of a PBLD protein or a gene encoding the PBLD protein in the exosomes; and
e) comparing the expression levels of the protein or the gene encoding the protein in the subject and the control group.

The term "control group" as used herein may mean a normal subject who has not developed squamous cell lung carcinoma, a non-squamous cell lung carcinoma patient group, a non-patient group, etc.

According to one aspect, when the expression level of the protein or the gene encoding the protein of the subject is higher than that of the control group, the subject may be judged to have developed squamous cell lung carcinoma or predicted to have a high risk of developing the squamous cell lung carcinoma. Desirably, when the expression level of the protein is 1.6 times higher than that of the control group, the subject may be judged to have developed squamous cell lung carcinoma or predicted to have a high risk of developing the squamous cell lung carcinoma.

According to one aspect, when the expression level of the PBLD protein or the gene encoding the PBLD protein of the subject is higher than that of the control group, the subject may be predicted to have a poor prognosis. The poor prognosis may be a relatively short recurrence-free survival period.

The expression level of the gene may be compared with that of the control group, but in another aspect, the expression level may be judged to be high when an exosome pbld concentration is higher than 90 pg/ml, and to be low when the exosome pbld concentration is lower than 90 pg/ml.

According to yet another embodiment of the present disclosure, there is provided a screening method of a squamous cell lung carcinoma therapeutic agent including:
a) treating a candidate substance to a biological sample, cell line, or non-human animal model isolated from a subject;
b) extracting exosomes from the biological sample, cell line or non-human animal model treated with the candidate substance; and
c) measuring the expression level of a PBLD protein or a gene encoding the PBLD protein in the extracted exosomes.

The "subject" has the same meaning as previously described herein. The "sample" may be as previously described herein, but may desirably be a tissue biopsy sample.

The "cell line" may desirably be a squamous cell lung carcinoma cell line.

The "animal model" may desirably be a squamous cell lung carcinoma animal model.

The measuring of the expression level of the PBLD protein or the gene encoding the PBLD protein may be performed as previously described herein.

According to one aspect, the screening method may further include, after the step,
d) selecting a candidate substance having a reduced expression level of the PBLD protein or the gene encoding the PBLD protein compared to a control sample that is not treated with the candidate substance.

The terms used in the embodiments are used for the purpose of description only, and should not be construed to be limited. A singular expression includes a plural expression unless the context indicates otherwise. In the present specification, it should be understood that term "including" or "having" indicates that features, numbers, steps, operations, components, parts or combinations thereof described herein are present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present application.

The present disclosure may have various modifications and various embodiments, and specific embodiments will be hereinafter illustrated in the drawings and described in detail in the detailed description. However, the present disclosure is not limited to specific embodiments, and it should be understood that the present disclosure covers all the modifications, equivalents and replacements within the idea and technical scope of the present disclosure. In describing the present disclosure, when it is determined that a detailed description of related known arts may obscure the gist of the present disclosure, the detailed description will be omitted.

### MODE FOR CARRYING OUT THE INVENTION

Hereinafter, embodiments will be described in detail with reference to the accompanying drawings. However, since various modifications may be made to the embodiments, the scope of the present disclosure is not limited or restricted by these embodiments. It should be understood that all modifications, equivalents and substitutes for embodiments are included in the scope of the present disclosure.

In addition, in the description with reference to the accompanying drawings, like components designate like reference numerals regardless of reference numerals and a duplicated description thereof will be omitted. In describing the embodiments, a detailed description of related known technologies will be omitted if it is determined that they unnecessarily make the gist of the embodiments unclear.

The present disclosure may have various modifications and various Examples, and specific Examples will be hereinafter illustrated in the drawings and described in detail in the detailed description. However, the present disclosure is not limited to specific Examples, and it should be understood that the present disclosure covers all the modifications, equivalents and replacements within the idea and technical scope of the present disclosure. In describing the present disclosure, when it is determined that a detailed description of related known arts may obscure the gist of the present disclosure, the detailed description will be omitted.

### Example 1. Collection of samples

### Example 1-1. Cell culture medium

Cell lines used in the present disclosure were HPAepiC, BEAS-2B, HSAEC1-KT, H1703, H2170, and SW900, and 2 × 10⁶ cells of each cell line were dispensed onto each 150 mm cell culture dish, and then the cells were grown to about 70 to 80% of the cell culture dish in a 36°C incubator. Thereafter, as a cell culture medium containing the FBS from which the exosomes had been removed, a culture medium cultured for 48 hours was obtained, and the cell debris was removed through centrifugation at 1,500 rpm for 5 minutes. Then, only the supernatant was recovered through centrifugation at 3,000 rpm for 30 minutes and stored in an ultra-low temperature freezer at - 80°C until the exosomes were extracted.

### Example 1-2. Blood sample

Whole blood samples collected in EDTA or SST tubes were centrifuged under conditions of 3,000 rpm, 30 minutes, and 4°C to recover plasma or serum, and then stored in an ultra-low temperature freezer at - 80°C until the exosomes were extracted.

### Example 2. Extraction of exosomes from samples

To extract exosomes from a cell culture medium, 200 ml of the culture medium was centrifuged at 5,000 rcf for 15 minutes using a 100 KDa Amicon Ultra 50 ml Filter tube and concentrated, and 0.5 ml of the concentrate was placed on a size-exclusion chromatography column, and then poured with PBS to obtain and use 6 to 9 fractions (0.5 ml per fraction, total 2 ml) of the exosome concentrate.

To extract exosomes from plasma, plasma stored in an ultra-low temperature freezer at - 80°C was centrifuged under conditions of 10,000 rcf, 30 minutes, and 4°C to remove precipitates. Thereafter, 0.5 ml of the plasma was placed on a size-exclusion chromatography column, and then poured with PBS to obtain and use 11 to 12 fractions (0.5 ml per fraction, total 2 ml) of the exosome concentrate.

### Example 3. Proteomic analysis

To discover exosome protein markers derived from squamous cell lung carcinoma cell lines, exosomes were extracted from three types of normal lung cells and three types of squamous epithelial cells and then proteomic analysis was performed. The concentrations of exosome proteins used for proteomic analysis were as shown in Table 1 below.

**[Table 1]**

| Cell line | Normal lung cell 1 | Normal lung cell 2 | Normal lung cell 3 | Squamous cell lung carcinoma cell 1 | Squamous cell lung carcinoma cell 2 | Squamous cell lung carcinoma cell 3 |
|---|---|---|---|---|---|---|
| Exosome Concentration (mg/ml) | 1.9 | 1.9 | 2.6 | 2.1 | 1.9 | 2 |

The peptide concentrations required for LC-MS analysis among the exosome proteins for proteomic analysis were measured using a tryptophan-based fluorescence assay method, and the concentrations were as shown in Table 2 below.

**[Table 2]**

| Exosome sample type | Peptide concentration (ug/uL) | Total peptide amount (ug) for TMT labeling |
|---|---|---|
| Normal lung cell 1 | 0.4346 | 30 |
| Normal lung cell 2 | 0.3587 | 30 |
| Normal lung cell 3 | 0.2246 | 30 |
| Squamous cell lung carcinoma cell 1 | 0.3328 | 30 |
| Squamous cell lung carcinoma cell 2 | 0.3810 | 30 |
| Squamous cell lung carcinoma cell 3 | 0.3625 | 30 |

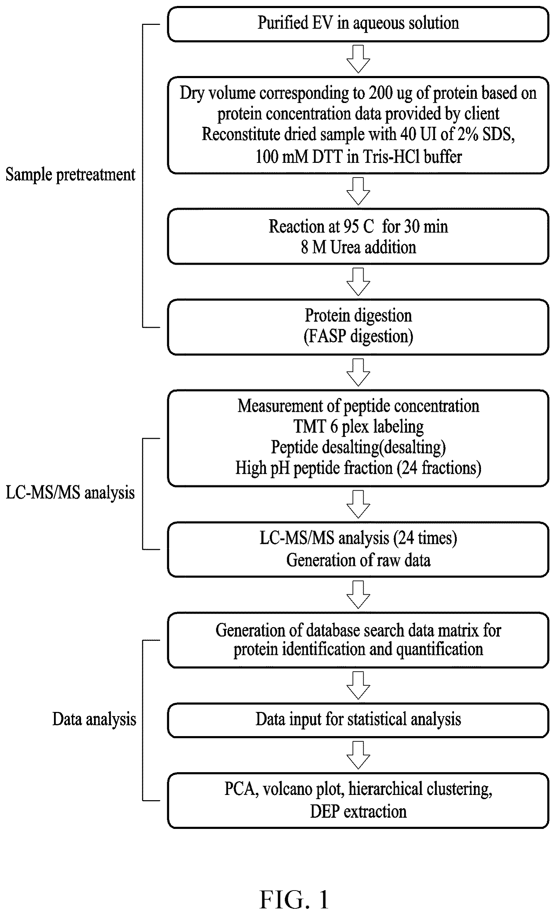

The proteomic analysis was performed as follows. A volume equivalent to 200 ug of an exosome sample derived from a squamous cell lung carcinoma cell line was taken, completely dried, dissolved in 2% SDS, and then protein pretreatment was performed using a FASP method. After protein digestion, each peptide sample was labeled with a Tandem Mass Tag (TMT 6 plex) reagent, combined, and then divided into 24 peptide fractions, and then subjected to NanoLC-MS analysis for 24 fractions. A schematic diagram of the process was shown in FIG. 1.

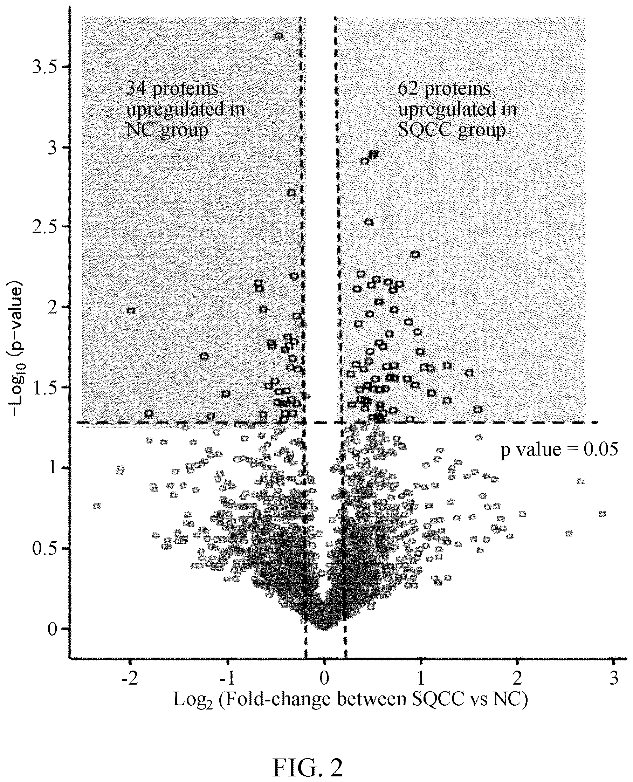

As a result of the analysis, a t-test was performed to determine a difference in protein expression levels between a control group and a squamous cell lung carcinoma group, and proteins that satisfied all criteria of p-value < 0.05 and fold change > 1.2 times or more were summarized into a volcano plot and shown in FIG. 2. As a result, it was confirmed that 62 proteins were upregulated in the squamous cell lung carcinoma group, and 34 proteins were upregulated in the control group. Among them, a phenazine biosynthesis-like domain-containing protein (PBLD, UniProt accession No. P30039) was identified as an exosome marker specific for squamous cell lung carcinoma.

### Example 4. Evaluation of diagnostic usability using PBLD

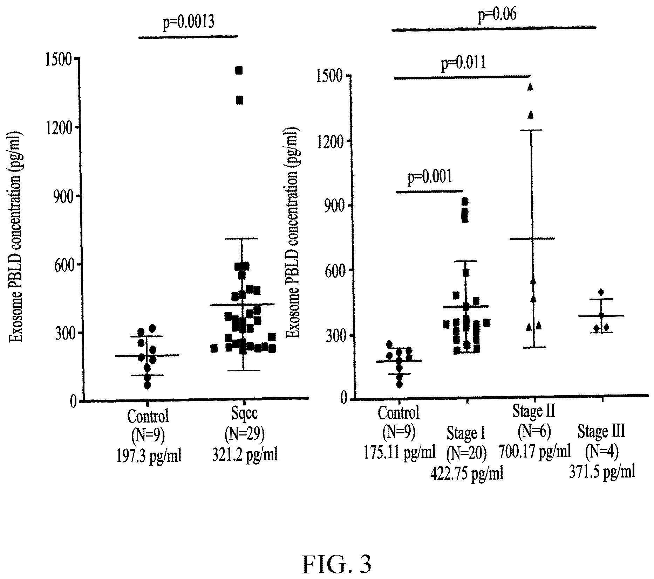

To evaluate the diagnostic usability of exosome PBLD for patients with squamous cell lung carcinoma, exosomes were extracted from the blood of 9 normal subjects and 29 patients with squamous cell lung carcinoma, and PBLD ELISA assay was performed, which was shown in FIG. 3. As an ELISA analysis result, it was confirmed that the PBLD concentration was 1.6 times (p = 0.0013) higher in the squamous cell lung carcinoma patient group than in the normal group, and the concentration was increased as a pathological stage increased.

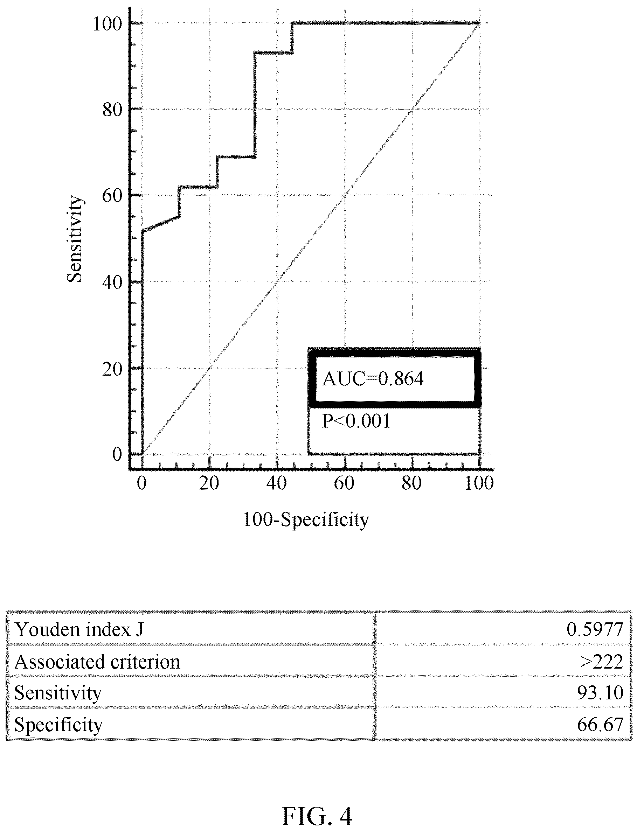

The results of ROC analysis using exosome PBLD were shown in FIG. 4, and the AUC value was 0.864, the sensitivity was 93.1%, and the specificity was 66.7%.

### Example 5. Comparative evaluation of diagnosis of lung adenocarcinoma and squamous cell lung carcinoma using PBLD

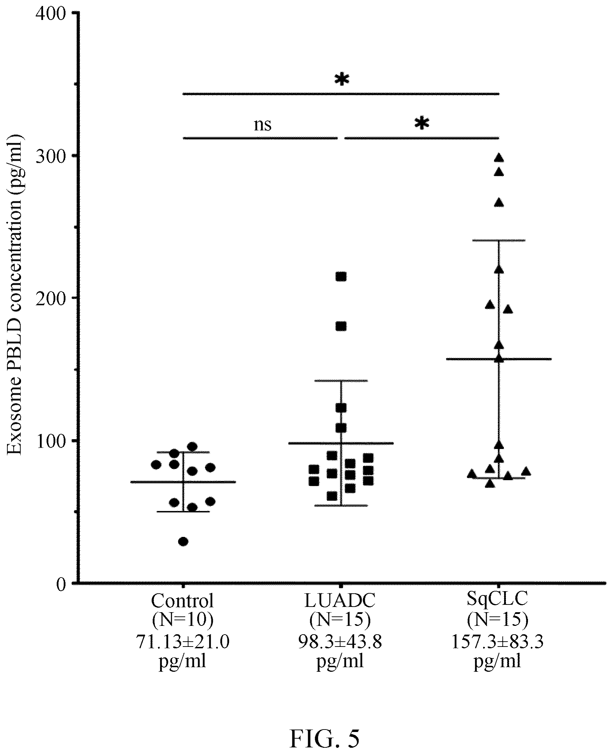

To evaluate the diagnostic usability of exosome PBLD in patients with lung adenocarcinoma and squamous cell lung carcinoma, exosome PBLD ELISA was performed on the blood of 10 normal subjects, 15 patients with lung adenocarcinoma, and 15 patients with squamous cell lung carcinoma, which was shown in FIG. 5. As an ELISA analysis result, it was confirmed that the concentration in the lung adenocarcinoma patient group was increased 1.4 times (p = 0.2164) compared to the normal group, but in the squamous cell lung carcinoma patient group, the concentration was increased 2.2 times (p = 0.0115).

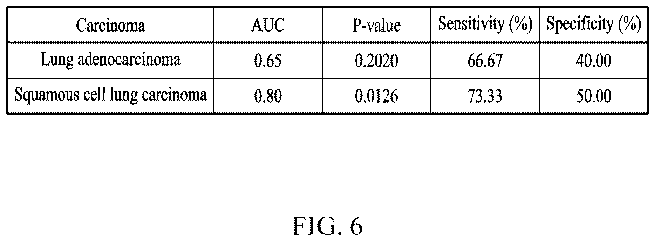

The results of ROC analysis were shown in FIG. 6, and it was confirmed that the AUC of exosome PBLD in lung adenocarcinoma was 0.65 (p = 0.2020), but the AUC in squamous cell lung carcinoma was 0.80 (p = 0.126), which was more significant in the diagnosis of squamous cell lung carcinoma.

### Example 6. Prognostic evaluation of squamous cell lung carcinoma using PBLD

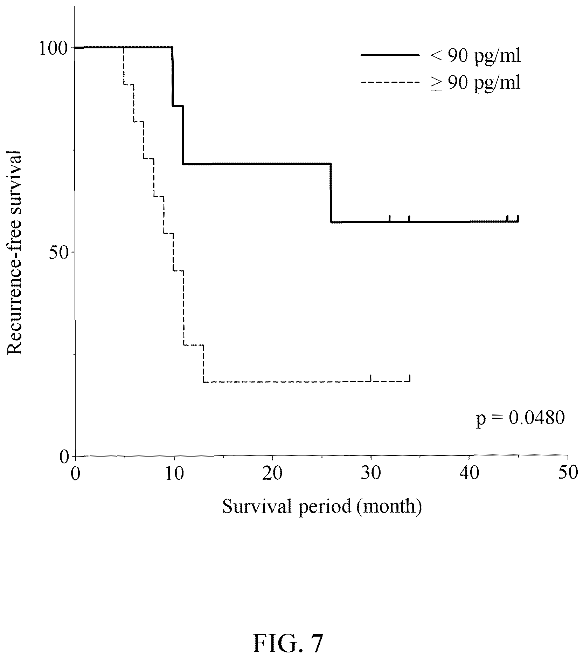

To evaluate the prognosis of squamous cell lung carcinoma patients using exosome PBLD, exosomes were extracted from the blood of 18 squamous cell lung carcinoma patients and PBLD ELISA assay was performed, and then Kaplan-Meier survival analysis was performed and a recurrence-free survival curve was shown in FIG. 7. As the recurrence-free survival analysis result, it was confirmed that the recurrence-free survival period of a patient group with a high concentration of exosome PBLD was about 10 months, which was significantly different (p = 0.048) from a patient group with a low concentrations (about 57 months).

As described above, although the embodiments have been described by the restricted drawings, various technical modifications and variations can be applied on the basis of the embodiments by those skilled in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result can be achieved.

Therefore, other implementations, other Preparation Examples, and equivalents to the appended claims fall within the scope of claims to be described below.

## Claims

1. A biomarker composition for diagnosis or prognosis of squamous cell lung carcinoma, comprising exosomes overexpressing a phenazine biosynthesis-like domain-containing protein (PBLD, UniProt accession No. P30039) or a gene encoding the PBLD protein.

2. A composition for diagnosis or prognosis of squamous cell lung carcinoma, comprising a preparation capable of measuring an expression level of a PBLD protein or a gene encoding the PBLD protein in exosomes.

3. The composition for diagnosis or prognosis of squamous cell lung carcinoma of claim 2, wherein the preparation capable of measuring the expression level of the protein is an antibody or an antigen-binding fragment thereof that specifically binds to the protein; or an aptamer that specifically binds to the protein.

4. The composition for diagnosis or prognosis of squamous cell lung carcinoma of claim 2, wherein the preparation capable of measuring the expression level of the gene is a primer or probe that specifically binds to a nucleic acid molecule of the gene.

5. A method for providing information for diagnosis or prognosis of squamous cell lung carcinoma, the method comprising:
a) extracting exosomes from a biological sample isolated from a subject; and
b) measuring an expression level of a PBLD protein or a gene encoding the PBLD protein in the extracted exosomes.

6. The method of claim 5, further comprising:
c) extracting exosomes from a biological sample isolated from a control group;
d) measuring an expression level of a PBLD protein or a gene encoding the PBLD protein in the exosomes; and
e) comparing the expression levels of the protein or the gene encoding the protein in the subject and the control group.

7. The method of claim 6, wherein when the expression level of the PBLD protein or the gene encoding the PBLD protein of the subject is higher than that of the control group, the subject is judged to have developed squamous cell lung carcinoma or predicted to have a high risk of developing the squamous cell lung carcinoma.

8. The method of claim 6, wherein when the expression level of the PBLD protein or the gene encoding the PBLD protein of the subject is higher than that of the control group, the subject is predicted to have a poor prognosis.

9. A kit for diagnosis of squamous cell lung carcinoma comprising the composition of any one of claims 2 to 4.

10. A screening method of a squamous cell lung carcinoma therapeutic agent, the screening method comprising:
a) treating a candidate substance to a biological sample, cell line, or non-human animal model isolated from a subject;
b) extracting exosomes from the biological sample, cell line or non-human animal model treated with the candidate substance; and
c) measuring an expression level of a PBLD protein or a gene encoding the PBLD protein in the extracted exosomes.

11. The screening method of claim 10, further comprising: after the step,
d) selecting the candidate substance having a reduced expression level of the PBLD protein or the gene encoding the PBLD protein compared to a control sample that is not treated with the candidate substance.
